# EUROPEAN PATENT SPECIFICATION

(11) **EP 2 371 331 B1**
(45) Date of publication and mention of the grant of the patent: **19.12.2012**
(21) Application number: 11160436.9
(22) Date of filing: 30.03.2011
(51) Int. Cl.: A61F 13/02, A61L 15/58, C09J 7/04

(54) **Stretchable pressure-sensitive adhesive bandage**
Dehnbare druckempfindliche Haftmittelbandage
Pansement adhésif étirable sensible à la pression

(30) Priority: 01.04.2010 JP 2010084744
(43) Date of publication of application: 05.10.2011
(73) Proprietor: Nitto Denko Corporation, Ibaraki-shi, Osaka (JP)
(72) Inventor: Kikuta, Noriyuki, Ibaraki-shi Osaka (JP); Hamada, Atsushi, Ibaraki-shi Osaka (JP); Suzuki, Seishi, Ibaraki-shi Osaka (JP)
(74) Representative: Grünecker, Kinkeldey, Stockmair & Schwanhäusser

(56) References cited:
- DE-A1- 2 733 549
- GB-A- 1 406 990
- GB-A- 2 010 933
- US-A- 4 654 254

## Description

### FIELD OF THE INVENTION

The present invention relates to a stretchable pressure-sensitive adhesive bandage for application mainly to medical instruments or skin surfaces. More particularly, the invention provides a stretchable pressure-sensitive adhesive bandage which is for use in the medical field, sanitary field, etc., and which can be used for fixing a gauze to a bent part or curved part, for fixing a medical instrument (e.g., fixing a catheter or fixing an oral intubation tube), for protecting the affected part against wetting, or for other purposes.

### BACKGROUND OF THE INVENTION

Generally, pressure-sensitive adhesive bandages for application to skin surfaces in the medical field, sanitary field, etc. are those obtained by forming a pressure-sensitive adhesive layer on a surface of a fabric substrate. For the purpose of restraining the bandaged part from moving, pressure-sensitive adhesive bandages employing a nonstretchable substrate are used. Meanwhile, for application to parts where the skin surface expands and contracts, such as bent parts and curved parts, pressure-sensitive adhesive bandages employing a stretchable substrate are used because of the necessity of fixing the bandaged part as well as moderately conforming to movements of the skin.

Most of the stretchable pressure-sensitive adhesive bandages that are actually on the market as the latter pressure-sensitive adhesive bandages employ a substrate which has no or slight stretchability in the width direction although stretchable in the length direction. When such a conventional stretchable pressure-sensitive adhesive bandage is applied to a medical instrument or skin surface, since the pressure-sensitive adhesive bandage is applied in the state of being moderately stretched in the length direction, reliable fixing can be attained on the basis of a balance between the stress caused by that property of the applied pressure-sensitive adhesive bandage by which the bandage recovers the original length and the adhesive force by which the bandage is adhesively fixed to the skin surface (see, JP-A-62-252495).

However, use of the pressure-sensitive adhesive bandage which is stretchable only in the length direction has the following problems. In the case of fixing a medical instrument such as, in particular, a catheter or an oral intubation tube with the pressure-sensitive adhesive bandage stretchable only in the length direction, the medical instrument must be fixed without fail and, at the same time, the bandage must moderately conform to movements of the skin surface and the mouth. However, the limited stretchability in the width direction inhibits the pressure-sensitive adhesive bandage from conforming to the movements and, as a result, there is a possibility that the bandage might peel off. Meanwhile, although the skin surface expands, contracts, or wrinkles to thereby alleviate the tensile stress caused by the pressure-sensitive adhesive bandage, since the skin of the bandaged part is restrained from moving by the less stretchable pressure-sensitive adhesive bandage, an excessive stress is imposed on the skin located around the bandaged part. During long-term wear, there is a fear that a rash or vesicles may occur on the skin.

### SUMMARY OF THE INVENTION

The present inventers diligently made investigations in order to overcome those problems of the conventional stretchable pressure-sensitive adhesive bandages. As a result, the inventors have found that the problems can be eliminated by using a fabric substrate which is stretchable in both the length direction and the width direction as the substrate of a stretchable pressure-sensitive adhesive bandage and by regulating the width-direction elongation thereof at a given load so as to be higher than the length-direction elongation thereof at the given load and further regulating the length-direction elongation thereof to a value within a relatively small specific range. The invention has been thus completed.

Namely, the invention provides:
a stretchable pressure-sensitive adhesive bandage, which comprises a fabric substrate, and a pressure-sensitive adhesive layer and a release liner superposed on one surface of the fabric substrate in this order,
the stretchable pressure-sensitive adhesive bandage being wound into a roll,
wherein when the release liner is removed from the stretchable pressure-sensitive adhesive bandage, the stretchable pressure-sensitive adhesive bandage has a width-direction elongation at a given load higher than a length-direction elongation at the given load, and has a length-direction elongation of 22 to 42% at a load of 0.5 N/5-mm width.

In an embodiment, when the release liner is removed from the stretchable pressure-sensitive adhesive bandage, the stretchable pressure-sensitive adhesive bandage has a width-direction elongation of 22 to 120% at a load of 0.5 N/5-mm width.

In another embodiment, when the release liner is removed from the stretchable pressure-sensitive adhesive bandage, the stretchable pressure-sensitive adhesive bandage has a width-direction elongation at break higher than a length-direction elongation at break.

In a still another embodiment, the fabric substrate is a knitted fabric.

In a still another embodiment, the fabric substrate comprises at least one fibers selected from the group consisting of polyester fibers, polyurethane fibers, nylon fibers, vinylon fibers, polyethylene fibers, polypropylene fibers, and cotton fibers. It is preferable that the fibers constituting the fabric substrate be at least one lowly water-absorbing fibers selected from the group consisting of polyester fibers, nylon fibers, vinylon fibers, polyethylene fibers, and polypropylene fibers. Among them, polyester fibers is especially preferred.

The stretchable pressure-sensitive adhesive bandage of the invention sufficiently conforms to omnidirectional movements of the bandaged skin surface because the width-direction elongation of the bandage at a given load is higher than the length-direction elongation thereof at the given load as well as the length-direction elongation thereof at a load of 0.5 N/5-mm width is regulated to 22 to 42%. The stretchable pressure-sensitive adhesive bandage is hence less apt to arouse skin troubles such as a rash and vesicles. Furthermore, when used for fixing a medical instrument such as a catheter or an oral intubation tube, the stretchable pressure-sensitive adhesive bandage can not only fix the instrument without fail but also moderately expand/contract and conform to movements of the skin surface and mouth. Consequently, the pressure-sensitive adhesive bandage produces an excellent effect that the bandage is less apt to peel off. Moreover, since the bandage employs a fabric substrate, the bandage has excellent air permeability and is less apt to arouse skin troubles. In the case where a fabric substrate obtained from lowly water-absorbing fibers is employed, water repellency can be imparted because the fibers themselves do not absorb water. Such a bandage is hence effective also in application to affected parts which are readily wetted by bathing, etc.

### BRIEF DESCRIPTION OF THE DRAWING

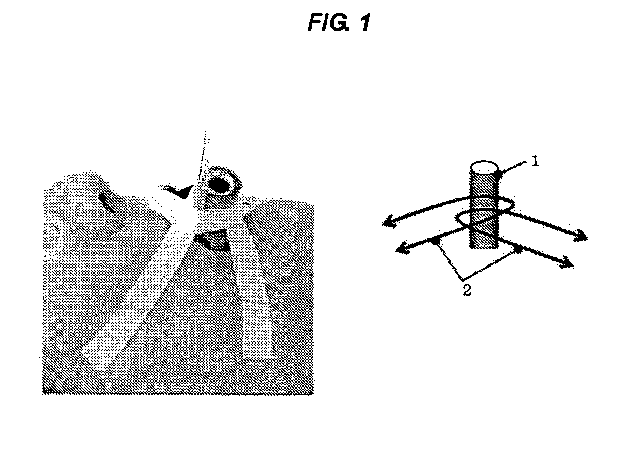

Fig. 1 is a view illustrating a method for fixing an intubation tube to a face model for oral intubation training using a stretchable pressure-sensitive adhesive bandage of the invention.

### Description of the Reference Numerals

- 1: Oral intubation tube (for training)
- 2: Stretchable pressure-sensitive adhesive bandage

### DETAILED DESCRIPTION OF THE INVENTION

The stretchable pressure-sensitive adhesive bandage of the invention is a bandage where a pressure-sensitive adhesive layer is formed on one surface of a fabric substrate and the surface of the pressure-sensitive adhesive layer is covered and protected with a release liner. The pressure-sensitive adhesive bandage has a long form, which has been wound into a roll.
The fabric substrate constituting the stretchable pressure-sensitive adhesive bandage of the invention is stretchable in both the length direction and the width direction. Unlike the pressure-sensitive adhesive bandages which have been supplied hitherto, the pressure-sensitive adhesive bandage of the invention is stretchable in both the length direction and the width direction, i.e., has omnidirectional stretchability, in which the width-direction stretchability is higher than the length-direction stretchability. Specifically, regarding the pressure-sensitive adhesive bandage configured of the fabric substrate and a pressure-sensitive adhesive layer formed on one surface thereof, i.e., the pressure-sensitive adhesive bandage from which the release liner is removed, the width-direction elongation thereof measured at a given load is higher than the length-direction elongation thereof measured at the given load, and the length-direction elongation thereof measured at a load of 0.5 N/5-mm width is 22 to 42%, preferably 22 to 30%. In case where the length-direction elongation thereof measured at a load of 0.5 N/5-mm width is less than 22%, application of this bandage to the skin tends to result in an enhanced stretched feeling that is accompanied by skin movements. In case where the length-direction elongation thereof measured at a load of 0.5 N/5-mm width exceeds 42%, this tends to result in a tube withdrawal length exceeding 10 mm. Such too low or too high length-direction elongations hence make it difficult to produce the effects of the invention.

In the invention, when the release liner is peeled off, the width-direction elongation of the pressure-sensitive adhesive bandage must be higher than the length-direction elongation thereof. It is specifically desirable that the width-direction elongation thereof as measured at a load of 0.5 N/5-mm width be in the range of 22 to 120%, preferably 22 to 80%.

Furthermore, also with respect to elongation as measured not at a given load but at break, it is preferred that the width-direction elongation at break be higher than the length-direction elongation at break. It is preferred that the elongation at break (maximum elongation) in the width direction (width-direction elongation at break) be regulated to a value in the range of about 200 to 600% and the elongation at break in the length direction (length-direction elongation at break) be regulated to a value in the range of about 100 to 200%. With respect to breaking strength, it is preferred that the pressure-sensitive adhesive bandage be regulated to have a width-direction breaking strength of 5 to 50 N/5-mm width and a length-direction breaking strength of 5 to 50 N/5-mm width, from the standpoints of durability concerning the load to be imposed on the pressure-sensitive adhesive bandage during wear and of handleability.

In this regard, elongations at a load of 0.5 N/5-mm width, elongations at break and breaking strengths of the bandage of the invention can be measured in accordance with the methods described in the column of "Examples" below. Further, elongations at a given load can also be measured in a similar manner as those for elongations at a load of 0.5 N/5-mm width. In addition, those properties of the bandage of the invention can be adjusted by appropriately selecting the material and fineness of the fibers constituting the fabric substrate, mode of knitting of the fabric substrate, and so on.

The fabric substrate in the stretchable pressure-sensitive adhesive bandage of the invention is not limited, and examples thereof include woven fabric, knitted fabric, and nonwoven fabric. Among them, it is preferred to use knitted fabric from the standpoint of imparting the specific stretchability which is characteristic to the invention. As the knitted fabric, use may be made of knitted fabric produced by warp knitting such as tricot knitting, raschel knitting, or Milanese knitting, weft knitting such as plain knitting or circular knitting, or the like. It is preferred to use weft knitting, of these, from the standpoint of regulating the width-direction stretchability so as to be higher than the length-direction stretchability.

The material of the warp and weft that constitute the fabric substrate is not particularly limited, and use may be made of fibers made of polyesters, polyurethanes, nylons, vinylon, polyethylene, polypropylene, cotton, etc. Among them, it is preferred to use polyester fibers, nylon fibers, vinylon fibers, polyethylene fibers, or polypropylene fibers, that are lowly water-absorbing fibers, from the standpoint of imparting water repellency to the pressure-sensitive adhesive bandage. It is especially preferred to use polyester fibers because of its low water-absorbing properties and high mechanical strength. One kind of fibers may be used solely or two or more kinds of the fibers may be used in combination.

With respect to the size of the fiber to be used as a warp or weft, it is desirable that the fineness thereof be 100 deniers or less, preferably 50 deniers or less, from the standpoint of the properties to be imparted to the fabric substrate, such as air permeability and stretchability. From the standpoints of retention of mechanical strength, moderate moisture-transporting ability, adhesion between the fabric substrate and the pressure-sensitive adhesive layer, etc., it is preferred that the fineness of the fiber be 5 deniers or more. The thickness of the fabric substrate is not limited. However, from the standpoints of handleability in application and of preventing the substrate from giving an uncomfortable feeling, it is desirable to regulate the thickness of the fabric substrate to about 10-150 µm, preferably about 20-75 µm.

The pressure-sensitive adhesive layer to be formed on one surface of the fabric substrate in producing the stretchable pressure-sensitive adhesive bandage of the invention not only has an adhesive force sufficient to adhesively fix a medical instrument to a skin surface but also has an adhesive force which enables the adhesive layer to sufficiently conform to the expansion/contraction of the fabric substrate and to movements of the skin surface. Specifically, it is desirable that the pressure-sensitive adhesive layer, when examined in a room-temperature adhesion test using a bakelite plate as an adherend, have an adhesive force of 2 to 6 N/20-mm width, preferably 3 to 5 N/20-mm width.

The pressure-sensitive adhesive which exhibits such an adhesive force is not particularly limited, and use may be made of acrylic pressure-sensitive adhesives, natural-rubber-based pressure-sensitive adhesives, synthetic-rubber-based pressure-sensitive adhesives, silicone type pressure-sensitive adhesives, vinyl ether type pressure-sensitive adhesives, or the like. Of these, acrylic pressure-sensitive adhesives are preferred because the pressure-sensitive adhesive properties thereof are easy to regulate and the acrylic pressure-sensitive adhesives have low skin-irritating properties, etc. Specifically, it is preferred to use an acrylic pressure-sensitive adhesive obtained by copolymerizing an alkyl ester of (meth)acrylic acid (alkyl (meth)acrylate) as a main monomer with at least one copolymerizable monomer and optionally subjecting the copolymer to chemical crosslinking with any of various crosslinking agents or physical crosslinking by irradiation with radiation such as electron beams or ultraviolet rays.

As the alkyl (meth)acrylate as a main monomer, an alkyl (meth)acrylate having an alkyl group with 2 to 12 carbon atoms can be used in an amount in the range of 40 to 99% by weight, preferably 40 to 60% by weight. As the copolymerizable monomer, use may be made of carboxyl-containing monomers such as (meth)acrylic acid, hydroxyl-containing monomers such as 2-hydroxyethyl (meth)acrylate, alkoxyl-containing monomers such as 2-methoxyethyl (meth)acrylate, monomers containing an acid amide group, such as 2-vinylpyrrolidone, and other monomers including vinyl acetate and monomers containing an amino group. One or more monomers suitably selected from such copolymerizable monomers according to purposes can be copolymerized in an amount in the range of 1 to 60% by weight.

It is preferred that the thickness of the pressure-sensitive adhesive layer to be formed on one surface of the fabric substrate be suitably determined while taking account of conformability to the skin, fixing properties, etc. It is, however, desirable to set the thickness thereof at, for example, about 10 to 180 µm, preferably about 15 to 150 µm, more preferably about 20 to 80 µm. The pressure-sensitive adhesive layer may be disposed on the entire surface of the fabric substrate, or may be partly disposed in a dot, streak, or pattern arrangement, etc. In the case where the pressure-sensitive adhesive layer is disposed in a streak arrangement, the shape of the streaks is not particularly limited so long as spaces functioning as air passages are ensured. Examples thereof include a linear shape, wavy shape, bell shape, serrate shape, and combinations of two or more thereof. Of these, a wavy shape is preferred from the standpoints of ensuring adhesion to the skin, preventing the end and edges from getting turned up, etc.

As mentioned above, the stretchable pressure-sensitive adhesive bandage of the invention is a bandage including a fabric substrate, a pressure-sensitive adhesive layer formed on one surface of the substrate, and a release liner superposed on the surface of the pressure-sensitive adhesive layer to cover and protect the pressure-sensitive adhesive surface, and the bandage is supplied after wound into a roll.

As the release liner, use may be made of a release liner for general use in, for example, medical pressure-sensitive adhesive tapes for application to the skin. Specifically, use may be made of a release liner obtained by coating a surface of wood-free paper, glassine paper, parchment paper, or the like with a release agent such as a silicone resin or a fluororesin. Also usable is a release liner obtained by coating a surface of a substrate with a release agent such as a silicone resin or a fluororesin, the substrate being wood-free paper on which a resin has been anchor-coated or to which a polyethylene resin or the like has been laminated.

In addition, printed lines may be provided on the release liner at given intervals (e.g., at intervals of 5 cm). This configuration is preferred because a bandage having such a release liner can be quickly cut into a necessary length with scissors or the like.

### Examples

The invention will be explained below in more detail by reference to an Example of the invention. Hereinafter, "%" means "% by weight".

### <Example and Comparative Examples>

The stretchable pressure-sensitive adhesive bandages shown in Table 1 were produced as an Example according to the invention and as Comparative Examples. Various properties of the fabric substrates to be used in the respective samples had been regulated by selecting the material of the fibers and the mode of knitting.

The stretchable pressure-sensitive adhesive bandages shown under the Example and Comparative Examples each were produced by forming a pressure-sensitive adhesive layer having a dry-basis thickness of 70 µm on the releasant-treated surface of a release liner, one surface of which had been treated with a release agent constituted of a silicone resin, and transferring the pressure-sensitive adhesive layer to one surface of the fabric substrate, followed by wound into a roll so that the release liner faced outward. Thus, the stretchable pressure-sensitive adhesive bandages were obtained as roll-shaped bandages.

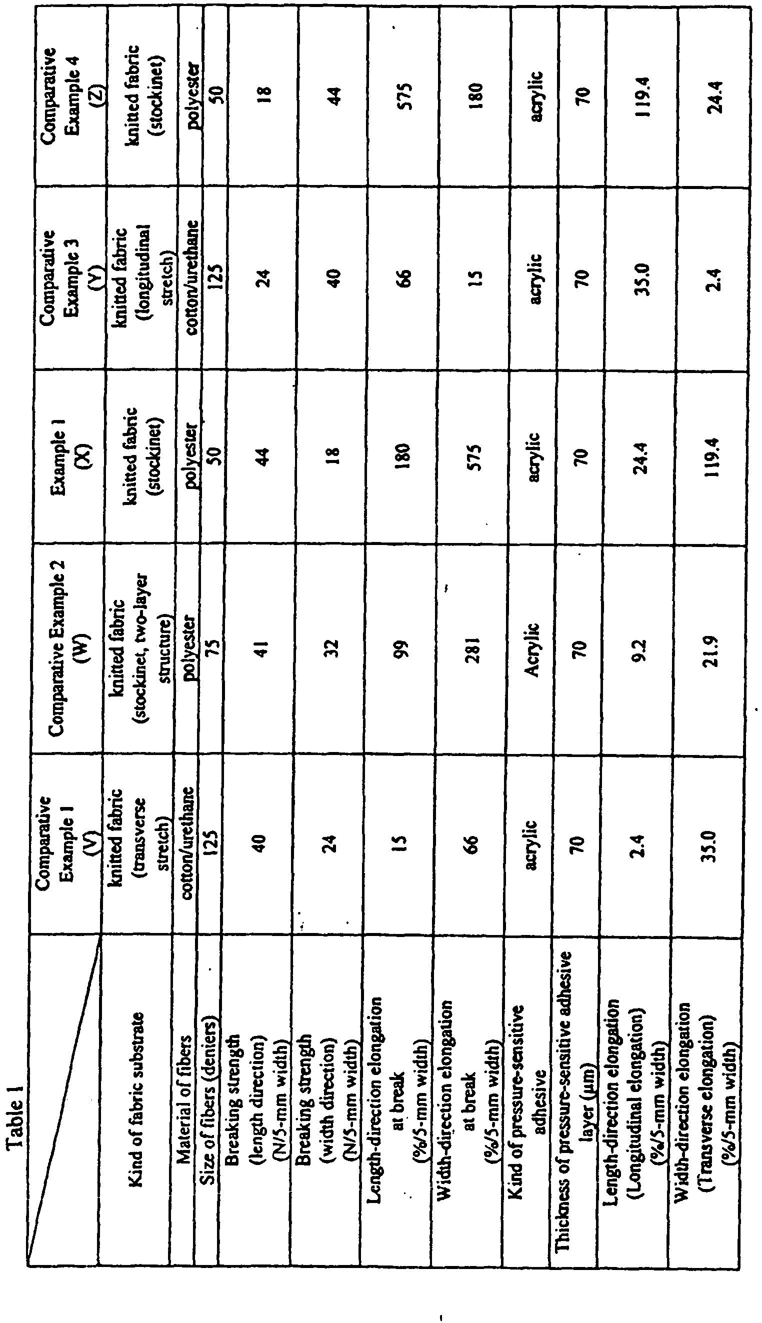

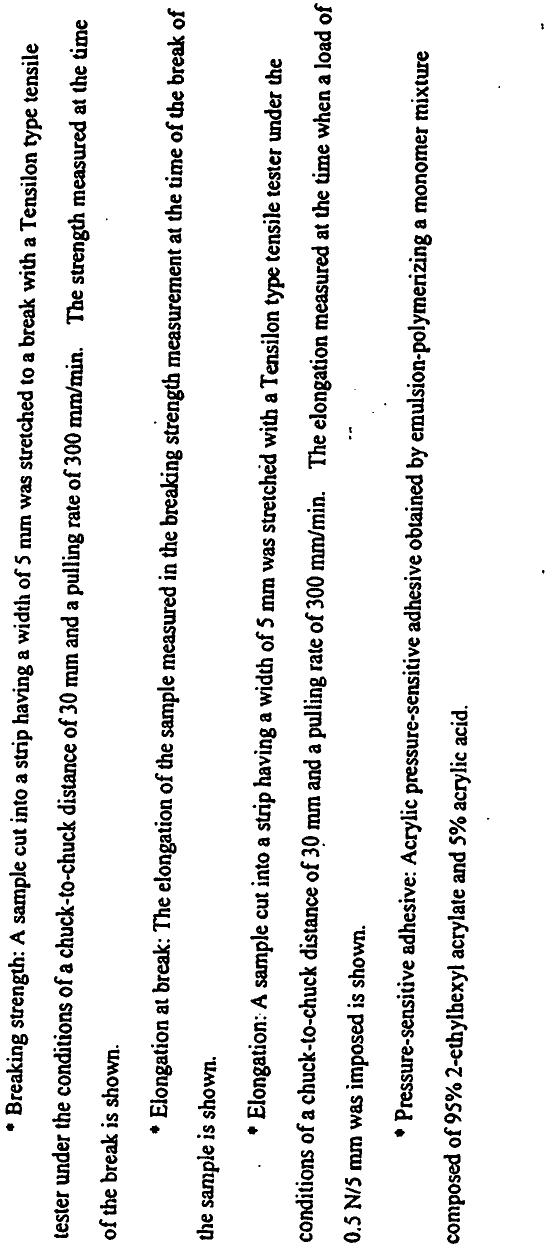

The stretchable pressure-sensitive adhesive bandages produced above as the Example and Comparative Examples were subjected to the following tests. The results obtained are shown in Table 2.

### <Medical-Instrument Fixing Property>

Each stretchable pressure-sensitive adhesive bandage was subjected to an oral-intubation-tube fixing test in which the bandage was applied to an oral intubation tube model as a medical instrument to fix the model as shown in Fig. 1.

Specifically, a tube having a diameter of 10 mm was inserted into the respiratory tract of the face model for oral intubation training shown in Fig. 1, and two strips of the stretchable pressure-sensitive adhesive bandage which had been cut into a width of 20 mm were wound around the tube and applied to the surface of the face model. Subsequently, the tube was withdrawn from the face model by hand, and the distance over which the tube was withdrawn before the pressure-sensitive adhesive bandage was stripped off (tube withdrawal length) was measured.

The case where the tube withdrawal length was 10 mm or less was rated as good (acceptable), and the case where the tube withdrawal length exceeded 10 mm was rated as poor (unacceptable).

### <Conformability to Skin>

Each stretchable pressure-sensitive adhesive bandage was applied to four male persons and examined for the degree to which the bandage conformed to expansion and contraction of the skin surface.

Specifically, the stretchable pressure-sensitive adhesive bandage (length, 50 mm; width, 20 mm) was applied to the outer surface of an elbow of each person so that the length direction of the bandage was the same as the flexing direction of the elbow. The bandage was applied while keeping the elbow stretched straight.

Subsequently, the elbow was flexed and evaluated for stretched feeling and uncomfortable feeling in five ratings: the case where the bandage gave a stretched feeling and an uncomfortable feeling was rated as point 1; the case where these feelings were generally not annoying was rated as point 3; and the case where the bandage gave neither a stretched feeling nor an uncomfortable feeling was rated as point 5. The points given by the four persons were averaged.

The case where the average of the evaluation points given by the four persons was point 3 or higher was rated as good (acceptable), and the case where the average thereof was less than point 3 was rated as poor (unacceptable).

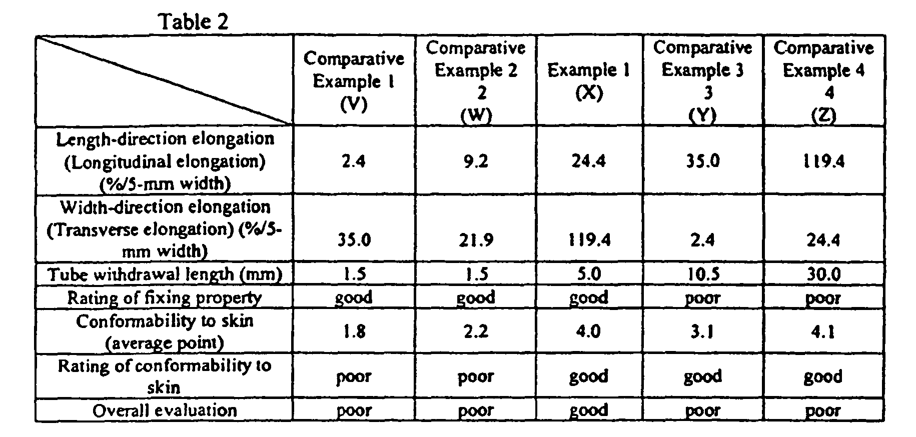

As the results given in Table 2, it is apparent that the bandage of the Example has a balanced combination of the property of fixing the intubation tube as a medical instrument and conformability to the skin, because the longitudinal-direction elongation (length-direction elongation) thereof has been set at a low value and the transverse-direction elongation (width-direction elongation) thereof has been set at a high value.

## Claims

1. A stretchable pressure-sensitive adhesive bandage, which comprises a fabric substrate, and a pressure-sensitive adhesive layer and a release liner superposed on one surface of the fabric substrate in this order,
the stretchable pressure-sensitive adhesive bandage being wound into a roll, wherein when the release liner is removed from the stretchable pressure-sensitive adhesive bandage, the stretchable pressure-sensitive adhesive bandage has a width-direction elongation at a given load higher than a length-direction elongation at the given load, and has a length-direction elongation of 22 to 42% at a load of 0.5 N/5-mm width.

2. The stretchable pressure-sensitive adhesive bandage according to claim 1, wherein when the release liner is removed from the stretchable pressure-sensitive adhesive bandage, the stretchable pressure-sensitive adhesive bandage has a width-direction elongation of 22 to 120% at a load of 0.5 N/5-mm width.

3. The stretchable pressure-sensitive adhesive bandage according to claim 1, wherein when the release liner is removed from the stretchable pressure-sensitive adhesive bandage, the stretchable pressure-sensitive adhesive bandage has a width-direction elongation at break higher than a length-direction elongation at break.

4. The stretchable pressure-sensitive adhesive bandage according to claim 1, wherein the fabric substrate is a knitted fabric.

5. The stretchable pressure-sensitive adhesive bandage according to claim 1, wherein the fabric substrate comprises at least one fibers selected from the group consisting of polyester fibers, polyurethane fibers, nylon fibers, vinylon fibers, polyethylene fibers, polypropylene fibers, and cotton fibers.

6. The stretchable pressure-sensitive adhesive bandage according to claim 1, wherein the fabric substrate comprises at least one lowly water-absorbing fibers selected from the group consisting of polyester fibers, nylon fibers, vinylon fibers, polyethylene fibers, and polypropylene fibers.

7. The stretchable pressure-sensitive adhesive bandage according to claim 1, wherein the fabric substrate comprises polyester fibers.

## Patentansprüche

1. Dehnbare druckempfindliche Haftmittelbandage umfassend ein Gewebesubstrat und eine druckempfindliche Haftmittelschicht und eine Schutzfolie (release liner), welche auf einer Oberfläche des Gewebesubstrats in dieser Reihenfolge angeordnet sind,
die dehnbare druckempfindliche Haftmittelbandage zu einer Rolle gewickelt ist, wobei, wenn die Schutzfolie von der dehnbaren druckempfindlichen Haftmittelbandage entfernt wird, die dehnbare druckempfindliche Haftmittelbandage eine höhere Dehnung in der Breite bei einer gegebenen Belastung als eine Dehnung in der Länge bei einer gegebenen Belastung aufweist, und eine Dehnung in der Länge von 22 bis 42 % bei einer Belastung von 0,5 N/5-mm Breite aufweist.

2. Dehnbare druckempfindliche Haftmittelbandage nach Anspruch 1, wobei, wenn die Schutzfolie von der dehnbaren druckempfindlichen Haftmittelbandage entfernt wird, die dehnbare druckempfindliche Haftmittelbandage eine Dehnung in der Breite von 22 bis 120 % bei einer Belastung von 0,5 N/5-mm Breite aufweist.

3. Dehnbare druckempfindliche Haftmittelbandage nach Anspruch 1, wobei, wenn die Schutzfolie von der dehnbaren druckempfindlichen Haftmittelbandage entfernt wird, die dehnbare druckempfindliche Haftmittelbandage eine höhere Reißdehnung in der Breite als Reißdehnung in der Länge aufweist.

4. Dehnbare druckempfindliche Haftmittelbandage nach Anspruch 1, wobei das Gewebesubstrat ein gestricktes Gewebe ist.

5. Dehnbare druckempfindliche Haftmittelbandage nach Anspruch 1, wobei das Gewebesubstrat wenigstens eine Faser aufweist, gewählt aus der Gruppe bestehend aus Polyesterfasern, Polyurethanfasern, Nylonfasern, Vinylonfasern, Polyethylenfasern, Polyproylenfasern und Baumwollfasern.

6. Dehnbare druckempfindliche Haftmittelbandage nach Anspruch 1, wobei das Gewebesubstrat wenigstens eine wenig Wasser absorbierende Faser umfasst, gewählt aus der Gruppe bestehend aus Polyesterfasern, Nylonfasern, Vinylonfasern, Polyethylenfasern und Polyproylenfasern.

7. Dehnbare druckempfindliche Haftmittelbandage nach Anspruch 1, wobei das Gewebesubstrat Polyesterfasern umfasst.

## Revendications

1. Pansement adhésif étirable sensible à la pression qui comprend un substrat en tissu et une couche adhésive sensible à la pression et un revêtement anti-adhésif superposé sur une surface dudit substrat en tissu, dans cet ordre,
le pansement adhésif étirable sensible à la pression étant enroulé en rouleau,
dans lequel lorsque le revêtement anti-adhésif est retiré du pansement adhésif étirable sensible à la pression, le pansement adhésif étirable sensible à la pression a un allongement dans le sens de la largeur à une charge donnée supérieure à l'allongement dans le sens de la longueur à la charge donnée, et a un allongement dans le sens de la longueur de 22 à 42 % à une charge de 0,5 N/5 mm de large.

2. Pansement adhésif étirable sensible à la pression selon la revendication 1, dans lequel lorsque le revêtement anti-adhésif est retiré du pansement adhésif étirable sensible à la pression, le pansement adhésif étirable sensible à la pression a un allongement dans le sens de la largeur de 22, à 120 % à une charge de 0,5 N/5 mm de large.

3. Pansement adhésif étirable sensible à la pression selon la revendication 1, dans lequel lorsque le revêtement anti-adhésif est retiré du pansement adhésif étirable sensible à la pression, le pansement adhésif étirable sensible à la pression a un allongement à la rupture dans le sens de la largeur supérieur à l'allongement à la rupture dans le sens de la longueur.

4. Pansement adhésif étirable sensible à la pression selon la revendication 1, dans lequel le substrat en tissu est un tissu tricoté.

5. Pansement adhésif étirable sensible à la pression selon la revendication 1, dans lequel le substrat en tissu comprend au moins une des fibres choisies dans le groupe comprenant les fibres de polyester, les fibres de polyuréthane, les fibres de nylon, les fibres de vinylon, les fibres de polyéthylène, les fibres de polypropylène et les fibres de coton.

6. Pansement adhésif étirable sensible à la pression selon la revendication 1, dans lequel le substrat en tissu comprend au moins une des fibres à faible absorption d'eau choisies dans le groupe comprenant les fibres de polyester, les fibres de nylon, les fibres de vinylon, les fibres de polyéthylène et les fibres de polypropylène.

7. Pansement adhésif étirable sensible à la pression selon la revendication 1, dans lequel le substrat en tissu comprend des fibres de polyester.
